Europäisches Patentamt

European Patent Office

Office européen des brevets

(19)

(11) Publication number: 0 241 042

A2

(12) **EUROPEAN PATENT APPLICATION**

(21) Application number: 87105327.8

(22) Date of filing: 10.04.87

(51) Int. Cl.⁴: **G01N 33/546** , //G01N15/14

(30) Priority: 11.04.86 JP 82099/86

(43) Date of publication of application:
14.10.87 Bulletin 87/42

(84) Designated Contracting States:
DE FR

(71) Applicant: **HITACHI, LTD.**
**6, Kanda Surugadai 4-chome**
**Chiyoda-ku Tokyo 101(JP)**

(72) Inventor: **Imai, Kyoko**
**c/o Makiguchi 622-22, Ichige**
**Katsuta-shi(JP)**
Inventor: **Nomura,Yasushi**
**991-15, Motoyoshidacho**
**Mito-shi(JP)**

(74) Representative: **Dost, Wolfgang, Dr. rer. nat.**
**Dipl.-Chem. et al**
**Patent- u. Rechtsanwälte Bardehle,**
**Pagenberg, Dost, Altenburg Frohwitter &**
**Partner Galileiplatz 1**
**D-8000 München 80(DE)**

(54) **A method for cell analysis.**

(57) An antibody reactive specifically with cells to be analyzed is attached to micro-capsules. The micro-capsules contain a fluorescent substance, a dye, a chelate complex, or a precursor thereof.

After a blood sample is smeared on a slide for microscope, cells to be analyzed in the smeared sample are immunologically bound to the antibody carrying the micro-capsules. Alternatively, instead of employing this method, it is also possible to bind cells to be analyzed in the sample to the antibody carrying the micro-capsules and then smear the sample on a slide for microscope.

Then, the slide preparation is observed under an optical microscope to detect micro-capsules combined with the cells to be analyzed. The micro-capsules are observed in an unbroken state and counted as the number of the corresponding cells to be analyzed.

# A METHOD FOR CELL ANALYSIS

## BACKGROUND OF THE INVENTION

This invention relates to a method for cell analysis, particularly a method which makes it possible to combine cells to be analyzed with a marker by utilizing the immunological competence of the cells and thereby detect the cells to be analyzed.

Methods for detecting cells by utilizing the immunological competence of blood cells such as lymphocyte and erythrocyte or tissue cells have begun to be applied and investigated very lately.

Lymphocytes include T cell (T lymphocyte) and B cell (B lymphocyte). Both of them are originated from a common marrow stem cell, and they function in cooperation with each other and cause immunoreaction in cooperation with macrophage, monocyte, multinuclear leukocyte, etc. In various immunological diseases, measurement of the abnormality in quantity of T cell playing an important role in cell-mediated immunity and B cell producing humoral antibody is said to be useful for diagnosis or grasping the pathology of the diseased.

As a first prior art showing a method for quantitating cells in blood, there is known Nippon Rinsho (Japanese Clinic), Vol. 40, Special Fall Number, p. 985 (1982). In this publication methods for quantitating T cells and B cells are described. In this first prior art, T cells are quantitated by utilizing their property of forming a rosette with sheep erythrocyte (SRBC) in a test tube. Alternatively, T cells are dyed by the membrane fluorescent antibody technique by use of an antibody reactive specifically to T cell (anti-T cell antibody). B cells are quantitated by utilizing their property of forming a rosette with mouse erythrocyte (MRBC) in a test tube. Alternatively, since T cell has immunoglobulin on the membrane surface, fluorescence-labeled anti-human immunoglobulin serum is applied and positive cells are observed under a fluorescence microscope (the membrane fluorescent antibody technique).

In the above-mentioned methods utilizing rosette formation reaction, much labor and a long time are required for quantitating T cells or B cells because of, for example, troublesome manual operations, a long time required for rosette formation reaction, and need for microscopic examination using a counting chamber. Furthermore, the membrane fluorescent antibody technique is disadvantageous in that the detection sensitivity is low because the number of molecules of a label compound (a fluorescent substance) which can be attached to one molecule of antibody is limited.

A second prior art is disclosed in U.S. Patent No. 4,510,244. This prior art discloses that an antigen having fluorescent particles bound thereto is combined with hybridoma cells, followed by introducing a liquid containing the resulting immune complex into a cell sorter to isolate individual fluorescence-labeled cells from unlabeled other cells. Even by this method, cells to be analyzed cannot be detected with sufficient sensitivity.

## SUMMARY OF THE INVENTION

The object of this invention is to provide a method for cell analysis which makes it possible to detect specific cells selectively with high sensitivity.

In accordance with this invention, cells to be analyzed are combined with an antibody cognate therewith carrying micro-capsules as a label, after which micro-capsules bound to the cells to be analyzed are detected, whereby the labeled cells to be analyzed are measured.

The label substance used in this invention is micro-capsules containing a substance having optical characteristics. This invention is characterized in that in the detection, the cells to be analyzed are detected without breakage of the micro-capsules.

In a preferred embodiment of this invention, plural types of cells to be analyzed can be detected for the same sample by using plural kinds of micro-capsules. In this case, different kinds of micro-capsules have antibodies cognate with different types of cells to be analyzed attached thereto and contain different substances having optical characteristics.

In this invention, a substance having optical characteristics which enables optical detection or a precursor thereof is encapsulated in micro-capsules, and the micro-capsules are combined with cells to be analyzed by utililizing the immunological competence of the cells, whereby discrimination of the cells is facilitated. Since a large amount of substance having optical characteristics can be encapsulated in micro-capsules, light emission or color production due to the optical substance and the like is increased when the micro-capsules are combined with cells, so that discrimination of cells to be analyzed from the surroundings (the background) is facilitated.

## DETAILED DESCRIPTION OF THE PREFERRED EMBODIMENT

In accordance with this invention an antibody reactive specifically with cells to be analyzed is attached to the micro-capsules, and the micro-capsules are combined with the cells by the antigen-antibody reaction. The term "antibody reactive specifically with cells to be analyzed" used herein means an antibody which is cognate with cells to be analyzed and reacts specifically with the cells to be analyzed themselves or a specific determinant on the surface of the cell to be analyzed.

As the micro-capsule, a lipid membrane such as liposome and the like can be suitably used but the micro-capsule is not limited thereto. Any micro-capsule may be used so long as it can contain a sufficient amount of a substance having optical characteristics or a precursor thereof. As the optical substance as indicator at the time of detection which is encapsulated in the micro-capsules, there can be used fluorescent substances, dyes, chelate complex compounds, precursors thereof, etc.

The fluorescent substances are preferably water-soluble ones such as fluorescein isothiocyanate isomer (FITC), carboxyfluorescein, phycoerythrin (PE), tetramethylrhodamine (TRITC), rhodamine, eosin (2',4',5',7'-tetrabromofluorescein) and acridine orangs (3,6-bis-dimethylaminoacridine).

As dyes and chelate complex compounds, there can be preferably used water-poluble dyes and water-soluble chelating agents such as complex of nitroso-PSAP and $Fe^{2+}$, eriochrom black T, 2-(2-thiazolyazo)-4-(methyl)-5-sulfomethyl-aminobenzoic acid (TAMSMB), tetramethylrhodamine isothiocyanate (RITC), rhodamine B, oxamine red, acid orange I, xylenol orange, methylxylenol blue, methylthymol blue, 5-Br-PAPS, 5-Br-PASS, dimethyl-sulfonazo-III, chromazurol B, nitro-PAPS and basophenanthroline.

As the precursors, pH indicators can be preferably used. In this case, color production, whereby cells to be analyzed can be detected, can be caused by changing the pH value of a suspension of the combined product of cells to be analyzed and microcapsules, without breaking the microcapsules.

However, certain fluorescent compounds cease to emit fluorescence owing to self-quenching in some cases when their concentration is high. Therefore, in encapsulating the such compound in the micro-capsules, its concentration is properly selected.

The micro-capsule is explained below by taking the case of liposome. Liposome is suitable for encapsulating therein a large amount of contents, as evident from the fact that it has heretofore been investigated as a drug carrier for missile treatment in the affected part. The number of substance having optical characteristics which can be encapsulated in such liposomes can be increased to $10^3$ to $10^4$ molecules per liposome. That is to say, when said number of the optical substance is converted to that per molecule of antibody attached to the liposome, the sensitivity is increased at least $10^3$ times at compared with the conventional methods.

Liposome can be prepared according to a conventional method described, for example, in Method in Enzymology, vol. 74, pp. 152-161.

When plural types of cells to be analyzed are present in a sample, this invention permits simultaneous quantitation of various types of cells. The constitution of this invention in this case is, for example, achieved by attaching a first antibody reactive specifically with first cells to be analyzed, to first micro-capsules containing a substance having optical characteristics or a precursor thereof; attaching a second antibody reactive specifically with second cells to be analyzed, to second micro-capsules containing a substance having optical characteristics or a precursor thereof; combining the first micro-capsules carrying the aforesaid first antibody and the second micro-capsules carrying the aforesaid second antibody with the corresponding cells to be analyzed, respectively; and optically detecting the first cells to be analyzed having the aforesaid first micro-capsules attached thereto and the second cells to be analyzed having the aforesaid second micro-capsules attached thereto, respectively. The micro-capsules can be supplied in the form of a suspension or in a dried state.

In the preferred embodiments of this invention, an optical microscope is used as a optical means of detection but this invention is not limited thereto. There can also be used a photometer of flow cytometer type constructed as follows: a liquid containing cells having an optical substance-containing micro-capsules attached thereto is passed continuously through a flow cell forming a narrow passage, and individual cells which pass through the flow cell successively are discriminated. When a flow cytometer such as that described in Howard et al., Practical Flow Cytometry, Alan R. Liss, Inc., l985, pp. 60. is used as a detector, the following procedure is carried out. A sample containing cells to be analyzed is mixed with an antibody carrying micro-capsules to combine the micro-capsules with cells to be analyzed, by immunoreaction. A suspension of the resulting immune complex is formed and then introduced into a flow cytometer, and individual micro-capsules are optically observed to count the number of cells to be analyzed. In this case, a fluorescent substance such as fluorescein isothiocyanate isomer (FITC) is encapsulated in the micro-capsules.

As cells to which the analysis method of this invention is applied, there can be thought of various cells, for example, the above-mentioned lymphocytes such as T cell and B cell, etc. These cells may be in the form of either a cell suspension or a blood or tissue preparation. As to the form of sample, there may be used either a blood smear formed on a slide or a bioptic preparation.

Several examples according to this invention are described below.

Example I

This example relates to the quantitation of T cells. It is one example of quantitation using an anti-human T cell antibody.

Preparation of micro-capsules:

Liposomes containing sphingomyelin and cholesterol were prepared. This preparation was carried out according to the conventional method described, for example, in Method in Enzymology, Vol. 74, pp. l52-l6l. A l00 $\mu$M solution of carboxyfluorescein as a fluorescent substance was encapsulated in the liposomes, and the surplus fluorescent substance was removed by dialysis. Next, the liposomes thus prepared were attached to commercially available anti-T cell antibody by use of SPDP (i.e., N-succinimidyl-3-(2-pyridyldithio)propionate) as a crosslinking agent.

In place of above carboxyfluorescein, there may be used other water-soluble ones mentioned hereinbefore.

Pretreatment of a sample:

Five milliliters of peripheral blood collected from a patient and incorporated with heparin was diluted 2-fold, and two volumes of the diluted sample was layered over one volume of Conray-Ficoll solution. After centrifugation at 400 g for 20 minutes, erythrocytes in the intermediate layer were collected and then washed three times, after which the number of cells was adjusted to 5 $\times$ l0$^5$/ml and monocytes of peripheral blood were separated. In a small test tube was placed 0.2 ml of a suspension of the monocytes separated and taken out, and centrifugated at l50 g for 5 minutes, and the supernatant was removed.

Analysis procedure:

With the monocyte suspension thus treated was sufficiently mixed 50 $\mu\ell$ of liposome-labeled anti-human T cell antibody obtained in the above preparation, and the resulting mixture was subjected to immunoreaction at 4°C for 60 minutes. The cells were washed three times with a 0.l M phosphate buffer solution (pH 7.2) containing l% bovine serum albumin, and the supernatant was removed, after which one drop of a glycerin buffer solution (glycerin l volume, a phosphate buffer solution l volume) and one drop of the cell suspension thus obtained were placed on a slide for microscope and covered with a cover glass. T cells were judged by means of a fluorescence microscope. The exciting wave length was 485 nm and the fluorescence wave length was 525 nm. The number of T cells to be analyzed contained in the sample was counted by counting the number of micro-capsules which emitted fluorescence.

The quantitation result obtained in this Example and that obtained by a conventional method are shown in Table I. The conventional method comprises binding an antibody carrying FITC molecules as a label to T cells, removing the unreacted cells by washing, placing a liquid containing the residual cells on a slide for microscope, and observing the same under a fluorescence microscope. Table I indicates that when this invention is applied, there can be attained a high sensitivity of I0 times or more that heretofore attained.

## Table 1 Results of T cell judgement

|  | Method of this invention | Conventional method |
| --- | --- | --- |
| Counted number of T cells | 430 | 38 |
| Reproducibility of judgement | CV 8% | CV 20% |

Example 2

This example relates to a method by which both T cells and B cells can be quantitated on the same sample as in Example I. In this Example, unlike in Example I, a sample was placed on a slide for microscope, after which cells to be analyzed were combined with micro-capsules.

Preparation of micro-capsules:

In the same manner as in Example I, there were prepared anti-T cell antibody having FITC-containing liposomes attached thereto and anti-B cell antibody having RITC-containing liposomes attached thereto.

Analysis procedure:

A thin-layer smear of the blood of a patient was prepared, dried, and immediately fixed. The fixation was conducted at 4°C by use of 85% ethanol for 5 minutes.

First, a liquid of the anti-T cell antibody having FITC-containing liposomes attached thereto was placed on the thus fixed blood smear, and the reaction was carried out at 37°C for 30 minutes. Then, the smear was sufficiently washed with a cold phosphate buffer solution. Subsequently, a liquid of the anti-B cell antibody having RITC-containing liposomes attached thereto was placed on the same smear and the reaction was carried out at 37°C for 30 minutes. Then, the smear was sufficiently washed with a cold phosphate buffer solution. After the reaction with the two antibodies, T cells and B cells were detected by means of a fluorescence microscope and the numbers thereof were individually counted. An exciting wave length of 480 nm and a fluorescence wave length of 520 nm were employed for counting the number of T cells, and an exciting wave length of 550 nm and a fluorescence wave length of 620 nm for counting the number of B cells. The results obtained are shown in Table 2.

### Table 2  Percentages (%) of T cells and B cells of periferal blood

| T cells | | B cells | |
|---|---|---|---|
| Method of this invention | E-RFC method | Method of this invention | M-RFC method |
| 78 | 70 | 11 | 9 |

In Table 2, the term "E-RFC method" refers to the sheep erythrocyte rosette formation reaction method which is a conventional method, and the term "M-RFC method" refers to the mouse erythrocyte rosette formation reaction method which is another conventional method. The conventional rosette formation reaction methods are disadvantageous in that although high in accuracy of quantitation, they require a long period of time for treatment and troublesome procedure. On the other hand, according to this invention, such disadvantages can be removed.

Example 3

In this Example, the numbers of leukocytes, erythrocytes and platelets were individually counted for the same sample as in Example I and their classification was made possible.

Preparation of micro-capsules:

Liposomes containing dyes different from one another in color tone were prepared. A solution (absorption wave length: 760 nm) of a complex of nitroso-PSAP and $Fe^{2+}$ was encapsulated in first liposomes for quantitating leukocytes. An eriochrom black T solution (absorption wave length: 550 nm) was encapsulated in second liposomes for quantitating erythrocytes. A TAMSMB solution (absorption wave length: 485 nm) was encapsulated in third liposomes for quantitating platelets. Subsequently, anti-human leukocyte antibody was attached to the first liposomes, anti-human erythrocyte antibody to the second liposomes, and anti-human platelet antibody to the third liposomes.

In place of above dyes and chelating agents, there may be used other water-soluble dyes and water-soluble chelating agents mentioned hereinbefore.

Analysis procedure:

In the same manner as in Example I, a blood sample of a patient was mixed successively with individual antibodies carrying various liposomes to react the antibodies with the corresponding cells to be analyzed. Then, the resulting cell suspension was placed on a slide for microscope to form a preparation. Alternatively, in the same manner as in Example 2, a blood sample was smeared on a slide for microscope, after which individual antibodies having various liposomes were successively allowed to act on the smeared sample to form a preparation.

The preparation formed was loaded on an analyzer equipped with an optical microscope such as that shown in U.S. Patent No. 4,362,386, and the labeled cells to be analyzed (of plural types) were observed. The numbers of cells of individual types were counted using the corresponding markers composed of liposomes as an indicator, and the blood cells were classified. The quantitation wave lengths were 760 nm, 550 nm and 485 nm and the signals at the respective wave lengths were individually treated.

According to this invention, cells can be quantitated with high sensitibity by a relatively simple procedure by combining microcapsules containing a substance having optical characteristics with cells to be analyzed, and therefore this invention can make a great contribution to examination work in hospitals, etc.

## Claims

1. A method for cell analysis comprising combining cells to be analyzed with an antibody cognate therewith carrying a label, and then detecting the cells to be analyzed combined with said antibody; characterized in that

the label of said antibody is micro-capsules containing a substance having optical characteristics, and

in said detection, the micro-capsules bound to the cells to be analyzed are optically detected without breaking said micro-capsules.

2. The method for cell analysis according to Claim I, characterized in that said micro-capsules are of plural kinds, and different kinds of micro-capsules have antibodies cognate with different types of cells to be analyzed attached thereto and contain different substances having optical characteristics.

3. The method for cell analysis according to Claim 2, characterized in that in the above-mentioned detection, the number of micro-capsules is counted for each kind thereof.

4. The method for cell analysis according to Claim I, characterized by placing a sample containing said cells to be analyzed on a slide for microscope, and then combining the cells to be analyzed in the sample on the slide with the antibody carrying the micro-capsules.

5. The method for cell analysis according to Claim I, characterized by mixing a sample containing said cells to be analyzed with the antibody carrying the micro-capsules, and then placing the resulting mixture on a slide for microscope.

6. The method for cell analysis according to Claim 4, or 5, characterized by using an optical microscope for the above-mentioned detection.

7. The method for cell analysis according to Claim I, characterized by forming a suspension of combined product of said cells to be analyzed and said antibody, and observing the suspension by means of a flow cytometer.

8. The method for cell analysis according to Claim I, characterized in that said substance having optical characteristics is selected from the group consisting of fluorescent substances, dyes, chelate complexes, and precursors thereof.